Europäisches Patentamt

European Patent Office

Office européen des brevets

(19)

(11) Veröffentlichungsnummer: **0 064 248**
**B1**

(12) # EUROPÄISCHE PATENTSCHRIFT

(45) Veröffentlichungstag der Patentschrift:
**19.09.84**

(21) Anmeldenummer: **82103481.6**

(22) Anmeldetag: **24.04.82**

(51) Int. Cl.³ **B 65 D 35/40**, B 65 D 1/32,
A 61 J 1/06

(54) **Mit einer Dosierkammer versehener Behälter.**

(30) Priorität: **30.04.81 DE 8112834 U**

(43) Veröffentlichungstag der Anmeldung:
**10.11.82 Patentblatt 82/45**

(45) Bekanntmachung des Hinweises auf die Patenterteilung:
**19.09.84 Patentblatt 84/38**

(84) Benannte Vertragsstaaten:
**BE DE IT NL SE**

(56) Entgegenhaltungen:
**CH - A - 440 548**
**DE - A - 2 827 227**
**GB - A - 1 465 383**
**US - A - 3 303 847**
**US - A - 3 917 120**

(73) Patentinhaber: **Hansen, Gerhard, Heerstrasse 130,
D-7166 Sulzbach-Laufen (DE)**

(72) Erfinder: **Hansen, Gerhard, Heerstrasse 130,
D-7166 Sulzbach-Laufen (DE)**

(74) Vertreter **Patentanwälte Phys. Bartels, Dipl.-Ing. Fink
Dipl.-Ing. Held, Lange Strasse 51,
D-7000 Stuttgart 1 (DE)**

# Beschreibung

Die Erfindung bezieht sich auf eine Packung entsprechend dem Oberbegriff des Anspruches 1.

Bei einer bekannten Packung der vorgenannten Art befindet sich zwischen der Dosierkammer und dem zusammendrückbaren Behälterteil eine Engstelle, die eine Einschnürung, eine zahnkranähnliche oder sternförmige Lamellenanordnung oder dgl. sein kann. Die Engstelle ist derart ausgebildet, daß das flüssige Medium durch eine Schleuderbewegung die Engstelle überwindend in die Dosierkammer gebracht werden kann. In der Praxis hat sich als nachteilig erwiesen, daß die Engstelle sehr genau ausgebildet sein muß, da schon bei verhältnismäßig kleinen, innerhalb üblicher Toleranzen liegender Änderungen des Querschnittes der Engstelle häufig Fehldosierungen auftreten (US-A-3 917 120).

Es ist auch bekannt, in eine Flasche ein Rohrstück einzustecken, das auf seiner ganzen Länge oder teilweise als Kapillare ausgebildet ist. Der Spalt zwischen dem Rohrstück und zwischen der Behälterwand oder einem auf den Behälter aufschraubbaren Deckel muß durch eine Dichtmanschette abgedichtet werden. Da Behälter, Rohrstück und Dichtmanschette jeweils für sich hergestellt und für den Gebrauch miteinander verbunden werden müssen, ist ein solcher Behälter verhältnismäßig teuer in seiner Herstellung. Zum Füllen der Pipette muß eine Verbindung des flüssigen Mediums mit dieser Pipette hergestellt und diese durch leichten Druck auf den Behälter gefült werden.

Dieser Druck muß zum Verhindern des Rückfließens der in der Pipette befindlichen Menge an flüssigem Medium beim Zurückschwenken des Behälters aufrechterhalten bleiben. Nach dem Trennen des in der Pipette und des im Vorratsbehälter befindlichen flüssigen Mediums wird das in der Pipette befindliche Medium durch stärkeren Druck auf den Vorratsbehälter aus dieser ausgetrieben. Das Eigengewicht des in der Pipette befindlichen flüssigen Mediums wirkt im Sinne von dessen Ausfließen aus der Pipette nach außen, was jedoch eine Verringerung des Druckes des gasförmigen Mediums im Behälter zur Folge hätte, welche Druckverringerung dem Ausfließen aus der Pipette entgegensteht (CH-A-440 548).

Der vorliegenden Erfindung liegt die Aufgabe zugrunde, Fehldosierungen weitgehend zu verhindern. Diese Aufgabe wird durch die Merkmale im Kennzeichnungsteil des Anspruches 1 erfindungsgemäß gelöst. Wegen der besonderen Ausbildung der Dosierkammer und wegen des Wegfalls der Engstelle zwischen der Dosierkammer und dem zusammendrückbaren Behälterteil lassen sich genaue Dosiermengen in einfacher Weise erreichen. Außerdem ist die neue Ausgestaltung herstellungstechnisch einfacher.

Durch die Merkmale der Ansprüche 2 und 3 wird eine bessere Haftung des flüssigen Mediums in der Dosierkammer erreicht.

Weitere Vorteile ergeben sich aus der Beschreibung und der Zeichnung. In dieser sind Behälter zur Aufnahme eines flüssigen Arzneimittels als Ausführungsbeispiel des Gegenstandes der Erfindung schematisch dargestellt. Es zeigt

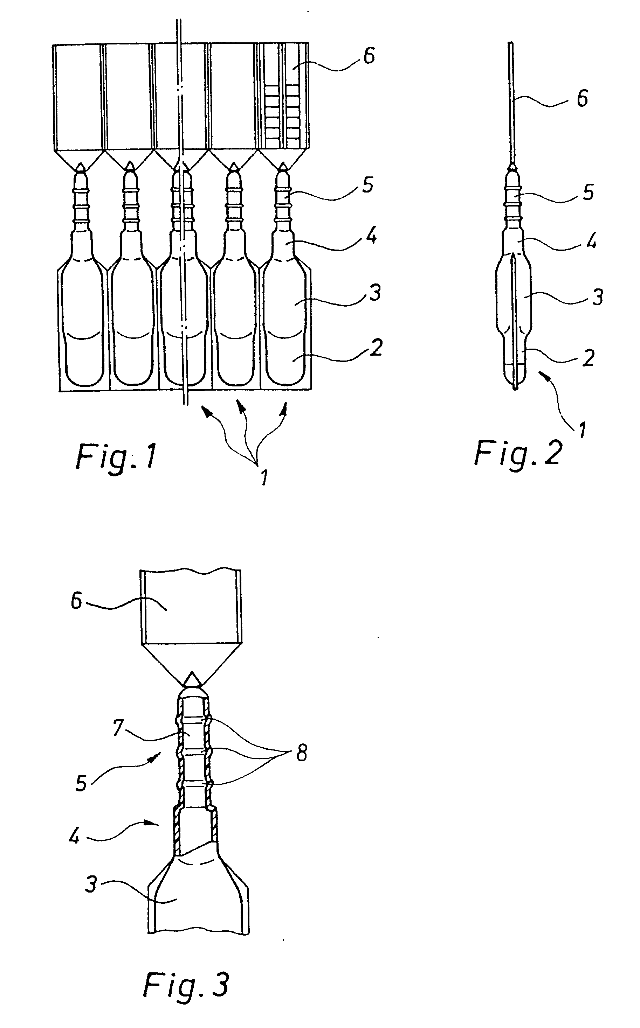

Fig. 1 eine Vorderansicht mehrerer zusammen hergestellter Behälter etwa in richtiger Größe,

Fig. 2 eine Seitenansicht,

Fig. 3 einen Ausschnitt mit Teillängsschnitt in größerem Maßstab.

Jeder Behälter 1 besteht aus einem elastischen, durchsichtigen Werkstoff. Jeweils zehn Behälter werden gemeinsam in einer Blasformmaschine hergestellt, mit einem Arzneimittel etwa bis zur Hälfte, vorzugsweise nicht ganz zur Hälfte, gefüllt und anschließend verschlossen. Der Rest des Behälters ist mit Luft gefüllt. Der Behälter hat einen unteren Bodenteil 2, einen mittleren, vorstehenden, flachen und zusammendrückbaren Behälterteil 3, einen Behälterhals 4, einen Dosierteil 5 und einen an den Dosierteil 5 an der vom Bodenteil 2 abgewandten Seite angesetzten Brechverschluß 6.

Der Dosierteil 5 hat eine kappilarenartig ausgebildete Dosierkammer 7, die mit im wesentlichen gleichem Durchmesser kanülenförmig gestaltet ist. Der Durchmesser der Dosierkammer 7 hängt von der Viskosität des in dem Behälter 1 befindlichen Arzneimittels ab. Die Oberflächenspannung des Arzneimittels muß so groß sein, daß sich die in der Dosierkammer 7 befindliche Menge darin hält und nicht zurückfließt, wenn der Behälter 1 in der in der Zeichnung dargestellten Lage gehalten wird, in welcher sich der Brechverschluß 6 oben befindet. In der Dosierkammer 7 sind in gleichen axialen Abständen die Dosierkammer 7 nach außen geringfügig erweiternde Ringrillen 8 angeordnet, wodurch eine bessere Haftung des Arzneimittels in der Dosierkammer 7 erreicht wird.

Der Innendurchmesser des Halsteiles 4 erweitert sich sprungartig auf der vom Brechverschluß 6 abgewandten Seite der Dosierkammer 7 und hat einen solchen Durchmesser, daß im Behälterhals 4 befindliches Arzneimittel in den Bodenteil 2 oder den Behälterteil 3 zurückfließen kann, wenn der Behälter 1 die in der Zeichnung dargestellte Lage einnimmt.

Der Behälter 1 nach dem Ausführungsbeispiel hat ein Volumen von etwa 1,0 ml und enthält als Arzneimittel Nasentropfen in einer Menge von etwa 0,5 ml. Das Volumen der Dosierkammer 7 beträgt 0,15 ml, so daß mindestens zwei dosierte Mengen z. B. für das linke und das rechte Nasenloch entnommen werden können.

Als Arzneimittel können auch Ohren- und Augentropfen oder dgl. abgefüllt werden. Es ist auch möglich, in dem Behälter anderes Füllgut, z. B. Leim, unterzubringen.

Für die Anwendung des im Behälter 1 befindlichen Arzneimittels oder dgl. wird dieser mit dem Brechverschluß 6 nach unten gehalten und durch leichtes Schütteln wird die Dosierkammer 7 gefüllt. Dann wird von dem Behälter 1 der Brechverschluß 6 abgebrochen und der oben befindliche Dosierteil 5 in ein Nasenloch eingeführt. Durch Ausüben eines Druckes auf den Behälterteil 3 wird die in der Dosierkammer 7 befindliche Menge an Arzneimittel in eines der Nasenlöcher gedrückt. Anschließend wird der Behälter 1 mit dem Dosierteil 5 nach unten gehalten und die Dosierkammer 7 durch leichtes Schütteln nochmals mit einer bestimmten Arzneimittelmenge gefüllt. Nach dem Drehen des Behälters 1 wird der Dosierteil 5 in das andere Nasenloch eingeführt und durch Drücken auf den Behälterteil 3 wird das in der Dosierkammer 7 befindliche Arzneimittel in das andere Nasenloch eingebracht.

## Patentansprüche

1. Packung, bestehend aus einem Behälter (1) und darin eingeschlossenem flüssigem und gasförmigen Medium, wobei der Behälter aus elastischem Kunststoff besteht und einen zusammendrückbaren Behälterteil (2, 3, 4) sowie eine in dessen Achsrichtung verlaufende kanülenförmig gestaltete Dosierkammer (7) aufweist, in welcher das flüssige Medium gehalten wird, auch wenn sie sich oberhalb des Behälterteils (2, 3, 4) befindet, wobei das Verhältnis der beiden Medien derart gewählt ist, daß beim Zusammendrücken des Behälterteils (2, 3, 4) in der Dosierkammer (7) befindliches flüssiges Medium aus dieser durch eine darin anbringbare Öffnung durch das gasförmige Medium austreibbar ist, dadurch gekennzeichnet, daß die Dosierkammer (7) als Kappilare ausgebildet ist, und daß sich der Behälter (1) unmittelbar an die Dosierkammer (7) anschließend über den Durchmesser der Kappillare hinaus sprungartig in den Behälterteil (2, 3, 4) erweitert.

2. Packung nach Anspruch 1, dadurch gekennzeichnet, daß die Dosierkammer (7) mindestens eine nach außen vorstehende Ringrille (8) hat.

3. Packung nach Anspruch 2, dadurch gekennzeichnet, daß in der Dosierkammer (7) mehrere Ringrillen (8) in axialen Abständen angeordnet sind.

## Claims

1. A pack consisting of a container (1) and a liquid medium and a gaseous medium enclosed therein, the container consisting of an elastic plastics material and having a compressible container portion (2, 3, 4) as well as a cannulashaped dosing chamber (7) which extends in the axial direction of the latter and in which the liquid medium is retained even if it is located above the container portion (2, 3, 4), the ration of the two media having been chosen in such a way that when the container portion (2, 3, 4) is being compressed the liquid medium contained in the dosing chamber (7) is expellable therefrom by the gaseous medium through an opening providable therein, characterized in that the dosing chamber (7) is designed as a capillary tube, and in that the container (1), directly adjoining the dosing chamber (7), abruptly widens, beyond the diameter of the capillary tube, into the container portion (2, 3, 4).

2. A pack as claimed in claim 1, characterized in that the dosing chamber (7) has at least one outwardly projecting annular groove (8).

3. A pack as claimed in claim 2, characterized in that several annular grooves (8) are provided in the dosing chamber (7) at axial intervals.

## Revendications

1. Emballage constitué par un récipient (1) et des milieux liquide et gazeux, y enfermés, le récipient étant constitué d'une substance synthétique élastique et présentant une partie de récipient compressible (2, 3, 4), ainsi qu'un compartment de dosage (7) façonné en forme de canule s'étendant suivant sa direction axiale, dans lequel le milieu liquide est maintenu, même lorsqu'il se trouve au-dessus de la partie de récipient (2, 3, 4), le rapport des deux milieux étant choisi de façon que, lors d'une compression de la partie de récipient (2, 3, 4), le milieu liquide se trouvant dans le compartiment de dosage (7) puisse être expulsé de ce dernier par le milieu gazeux, à travers un orifice pouvant être pratiqué dans le compartiment de dosage, caractérisé en ce que le compartiment de dosage (7) est réalisé sous la forme de capillaire et en ce que le récipient (1) s'élargit brusquement au-delà du diamètre du capillaire, en la partie de récipient (2, 3, 4), directement à la suite du compartiment de dosage (7).

2. Emballage suivant la revendication 1, caractérisé en ce que le compartiment de dosage (7) présente au moins une gorge annulaire (8) faisant saillie vers l'extérieur.

3. Emballage suivant la revendication 2, caractérisé en ce que plusieurs gorges annulaires (8) sont agencées à des distances axiales dans le compartiment de dosage (7).

*Fig.1*

*Fig.2*

*Fig.3*